(19)

Europäisches
Patentamt
European
Patent Office
Office européen
des brevets

(11)   **EP 3 572 812 A1**

(12)   # EUROPEAN PATENT APPLICATION

(43) Date of publication:
**27.11.2019  Bulletin 2019/48**

(51) Int Cl.:
***G01N 33/68*** *(2006.01)*

(21) Application number: **18174414.5**

(22) Date of filing: **25.05.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Bayer Aktiengesellschaft**
  **51373 Leverkusen (DE)**
• **Bayer Pharma Aktiengesellschaft**
  **13353 Berlin (DE)**

(72) Inventors:
• **Schulz, Anke**
  **10585 Berlin (DE)**

• **Machens, Kathrin**
  **13505 Berlin (DE)**
• **Hilpert, Jan**
  **10115 Berlin (DE)**
• **Schmitz, Heinz**
  **10715 Berlin (DE)**
• **Tetzner, Reimo**
  **10439 Berlin (DE)**
• **Gashaw, Isabella**
  **13503 Berlin (DE)**

(74) Representative: **BIP Patents**
  **c/o Bayer Intellectual Property GmbH**
  **Alfred-Nobel-Straße 10**
  **40789 Monheim am Rhein (DE)**

(54)   **DIAGNOSIS OF ENDOMETRIOSIS BY DECREASED PROTEIN LEVELS OF BETA2-MICROGLOBULIN PROTEIN (B2M) AND ONE OR MORE FURTHER BIOMARKERS**

(57)   The present application relates to a method for diagnosis of endometriosis in a subject, comprising the steps of
(i) determining the level of $\beta$2-Microglobulin protein ($\beta$2M) as a biomarker in a sample of said subject to be diagnosed;
(ii) comparing the biomarker level as determined in steps (i) to a respective control level derived from one or more subject without endometriosis;
wherein a reduced level as compared to the respective control level of $\beta$2M is indicative for the presence of endometriosis in said subject to be diagnosed. In particular, the application relates to a diagnostic classifier comprising a combination of a plurality of biomarkers selected from the group consisting of $\beta$2M protein level, CA19-9 level, CA125 protein level, PON-1 activity, MMP1 protein level and MMP2 protein level.

SEQUENCE LISTING

<110> Bayer AG
      Bayer Pharma AG

<120> Diagnosis of endometriosis by decreased protein levels of β2-Microglobulin protein (β2M) and one or more further biomarkers

<130> BHC 17 3 060 EP

<160> 1

<170> BiSSAP 1.3.6

<210> 1
<211> 355
<212> PRT
<213> human


<220>
<223> PON-1 protein sequence

<400> 1
Met Ala Lys Leu Ile Ala Leu Thr Leu Leu Gly Met Gly Leu Ala Leu
1               5                   10                  15
Phe Arg Asn His Gln Ser Ser Tyr Gln Thr Arg Leu Asn Ala Leu Arg
                20                  25                  30
Glu Val Gln Pro Val Glu Leu Pro Asn Cys Asn Leu Val Lys Gly Ile
                35                  40                  45
Glu Thr Gly Ser Glu Asp Leu Glu Ile Leu Pro Asn Gly Leu Ala Phe
            50                  55                  60
Ile Ser Ser Gly Leu Lys Tyr Pro Gly Ile Lys Ser Phe Asn Pro Asn
65                  70                  75                  80
Ser Pro Gly Lys Ile Leu Leu Met Asp Leu Asn Glu Glu Asp Pro Thr
                85                  90                  95
Val Leu Glu Leu Gly Ile Thr Gly Ser Lys Phe Asp Val Ser Ser Phe
                100                 105                 110
Asn Pro His Gly Ile Ser Thr Phe Thr Asp Glu Asp Asn Ala Met Tyr
            115                 120                 125
Leu Leu Val Val Asn His Pro Asp Ala Lys Ser Thr Val Glu Leu Phe
            130                 135                 140
Lys Phe Gln Glu Glu Glu Lys Ser Leu Leu His Leu Lys Thr Ile Arg
145                 150                 155                 160
His Lys Leu Leu Pro Asn Leu Asn Asp Ile Val Ala Val Gly Pro Glu
                165                 170                 175
His Phe Tyr Gly Thr Asn Asp His Tyr Phe Leu Asp Pro Tyr Leu Gln
            180                 185                 190
Ser Trp Glu Met Tyr Leu Gly Leu Ala Trp Ser Tyr Val Val Tyr Tyr
            195                 200                 205
Ser Pro Ser Glu Val Arg Val Val Ala Glu Gly Phe Asp Phe Ala Asn
            210                 215                 220
Gly Ile Asn Ile Ser Pro Asp Gly Lys Tyr Val Tyr Ile Ala Glu Leu
225                 230                 235                 240
Leu Ala His Lys Ile His Val Tyr Glu Lys His Ala Asn Trp Thr Leu
                245                 250                 255
Thr Pro Leu Lys Ser Leu Asp Phe Asn Thr Leu Val Asp Asn Ile Ser
                260                 265                 270

```
Val Asp Pro Glu Thr Gly Asp Leu Trp Val Gly Cys His Pro Asn Gly
        275             280             285
Met Lys Ile Phe Phe Tyr Asp Ser Glu Asn Pro Pro Ala Ser Glu Val
        290             295             300
Leu Arg Ile Gln Asn Ile Leu Thr Glu Glu Pro Lys Val Thr Gln Val
305             310             315             320
Tyr Ala Glu Asn Gly Thr Val Leu Gln Gly Ser Thr Val Ala Ser Val
        325             330             335
Tyr Lys Gly Lys Leu Leu Ile Gly Thr Val Phe His Lys Ala Leu Tyr
        340             345             350
Cys Glu Leu
        355
```

**Description**

**Technical Field of the Invention**

The present invention is in the field of medicine, in particular to the field diagnostics and prognosis of endometriosis. Furthermore, it relates to methods, means, kits and uses for diagnosis of endometriosis employing detection of levels of β2-Microglobulin protein (β2M) and at least one further biomarker.

**Background of the Invention**

Endometriosis is a debilitating gynecologic disease characterized by the presence of endometrial tissue outside the uterine cavity, most often found on the pelvic organs and structures like e.g. peritoneum, ovaries, and bowel. The prevalence of endometriosis in reproductive age women is 2-10% and as high as 35-50% in women with pain and/or unexplained infertility. Endometriosis is a major cause of disability and significantly compromised quality of life leading to a high economic burden.

Laparoscopy is the gold standard diagnostic test for endometriosis, despite the associated surgical risks. Mostly, laparoscopy is combined with surgical removal of endometriotic lesions.

The requirement for invasive surgery for the diagnosis of endometriotic lesions strongly contributes to an average latency of 7-11 years from onset of symptoms to definitive diagnosis. This delay in diagnosis and consequently endometriosis-specific treatment leads to more advanced disease, supporting progression of disease over time. On the other hand, only one third of women who undertake a laparoscopic procedure will receive a diagnosis of endometriosis; therefore many disease-free women are unnecessarily exposed to surgical risk as reported by Frishman GN and Salak JR. (J Minim Invasive Gynecol. 2006 Nov-Dec;13(6):546-58). Finally, the validity of laparoscopy as a reference test for endometriosis has been assessed as being highly dependent on the skills of the surgeon.

Currently, there are no non-invasive or minimally invasive diagnostic tests pursued in late clinical development or available in clinical practice. Many blood or serum parameters have been reported to be altered by endometriosis. However, none of these markers alone or in combination has been validated as being diagnostic. The lack of an *in vitro* diagnostic (IVD) has been recently confirmed by multiple reviews using Cochrane methods for blood (1) but also tissue based biomarkers (BMs) (2) and their combinations with imaging techniques for specific cohorts of patients (3, 4).

Molecular biomarkers specific for symptomatic women affected by endometriosis would set the basis for the development of non-invasive diagnostic tests and would lead to a shortening of the time to diagnosis as they could be performed in an outpatient setting and are not associated with the risks of laparoscopy.

Hence, there is a great need for such molecular biomarkers allowing non-invasive diagnostics of endometriosis.

The inventors have now found that levels of β2-Microglobulin protein (β2M) is well suited as such molecular biomarker for endometriosis, as its level is decreased in samples of endometriotic patients. Further, it has been found that if β2M combined with CA19-9 levels as a further biomarker, confidence can be enhanced. Further enhancement in this regard can be achieved if β2M alone or in combination with CA19-9 if one or more further biomarker from a group of CA125 protein, PON-1 activity, MMP1 protein, and MMP2 protein are comprised with them in a diagnostic classifier.

These biomarkers are associated with the presence of endometriotic lesions in symptomatic patients with endometriosis.

**Summary of the Invention**

The invention in one aspect relates to β2-Microglobulin protein (β2M) level as a biomarker for endometriosis as well as diagnostic classifiers comprising the same. Further the invention relates to β2-Microglobulin protein (β2M protein) level and CA19-9 levels as a biomarker for endometriosis as well as diagnostic classifiers comprising these. Further the one or more further biomarker selected from the group consisting of CA125 protein level, PON-1 activity, MMP1 protein level, and MMP2 protein level may be used in addition to β2-Microglobulin (β2M) protein level and CA19-9 levels as biomarkers for endometriosis or may be contained in the diagnostic classifier.

The inventors found that in principle decreased β2M protein levels in samples of a subject to be diagnosed as compared to a control level are indicative of endometriosis.

Accordingly, the present invention relates to a method for diagnosis of endometriosis in a subject, the method comprising the steps of

(i) determining the level of β2-Microglobulin protein (β2M protein) as a biomarker in a sample of said subject to be diagnosed;

(ii) comparing the biomarker level as determined in steps (i) to a respective control level derived from one or more subject without endometriosis;

wherein a reduced level as compared to the respective control level of β2M is indicative for the presence of endometriosis in said subject to be diagnosed.

The invention also relates to the use of β2-Microglobulin protein (β2M protein) binding antibody or an antigen binding peptide fragment thereof, and one or more antibodies or an antigen binding peptide fragment thereof binding one of more further biomarkers selected from the group consisting of CA125 protein, CA19-9, PON-1 activity, MMP1 protein, and MMP2 protein for the diagnosis of endometriosis or monitoring the response of a subject to the treatment for endometriosis.

The present invention further relates to research and/or diagnostic kit for the diagnosis of endometriosis, wherein the kit comprises β2-Microglobulin protein (β2M protein) binding antibody or an antigen binding peptide fragment thereof, and one or more antibodies or an antigen binding peptide fragment thereof binding one of more further biomarkers selected from the group consisting of CA125 protein, CA19-9, PON-1 activity, MMP1 protein, and MMP2 protein.

**Figures**

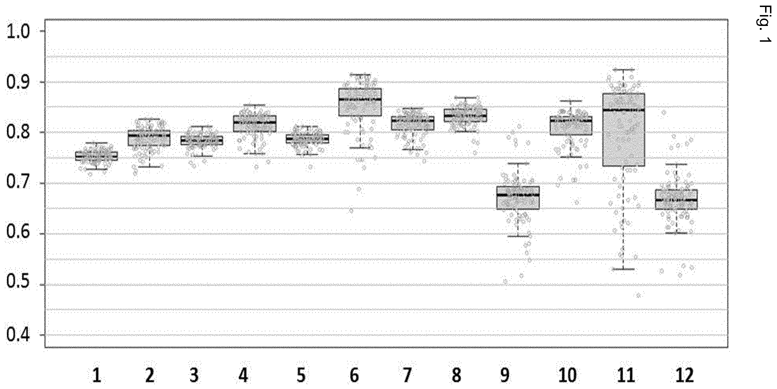

**Figure 1:** Boxplot display of cross validated (cv) area under the curve (AUC) values (y-axis) for biomarker combinations calculated from blood concentrations at day 1 to 4 of menstrual cycle. The number at the x-axis indicate the number of the combination as follows: β2M protein level and CA 19-9 level (combination 1); β2M protein level, CA-125 protein level and CA 19-9 level (combination 2); β2M protein level, PON-1 activity and CA 19-9 level (combination 3); β2M protein level, CA 19-9 level and MMP1 protein level (combination 4); β2M protein level, CA 19-9 level, PON-1 activity and MMP2 protein level (combination 5); β2M protein level, CA-125 protein level, CA 19-9 level and MMP1 protein level (combination 6); β2M protein level, CA-125 protein level, CA 19-9 level and MMP2 protein level (combination 7); β2M protein level, CA-125 protein level, CA 19-9 level and PON-1 activity (combination 8); β2M protein level, CA-125 protein level, CA 19-9 level, MMP1 protein level and MMP2 protein level (combination 9); β2M protein level, CA-125 protein level, CA 19-9 level, PON-1 activity and MMP2 protein level (combination 10); β2M protein level, CA-125 protein level, CA 19-9 level, PON-1 activity and MMP1 protein level (combination 11); β2M protein level, CA-125 protein level, CA 19-9 level, PON-1 activity, MMP1 protein level and MMP2 protein level (combination 12).

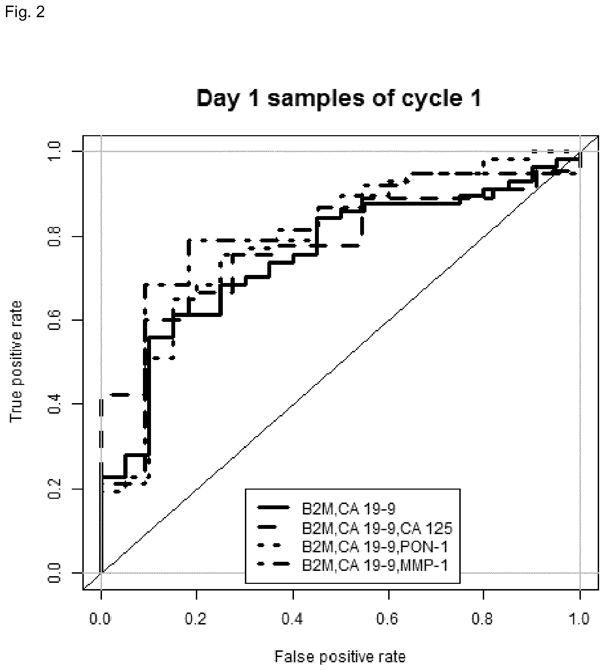

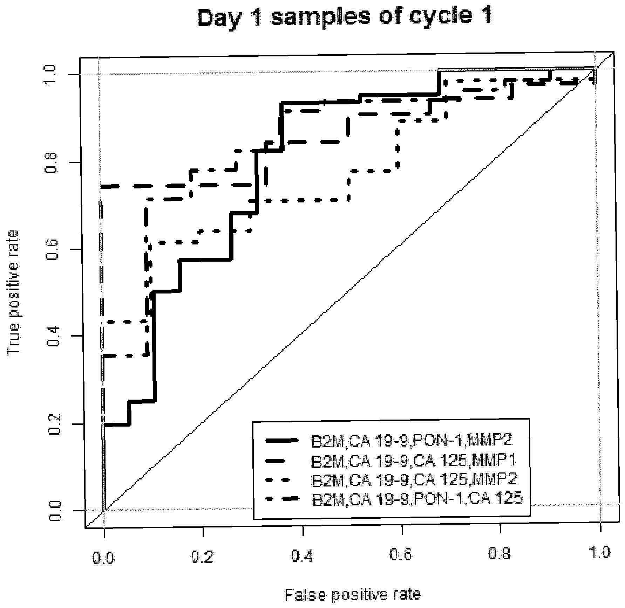

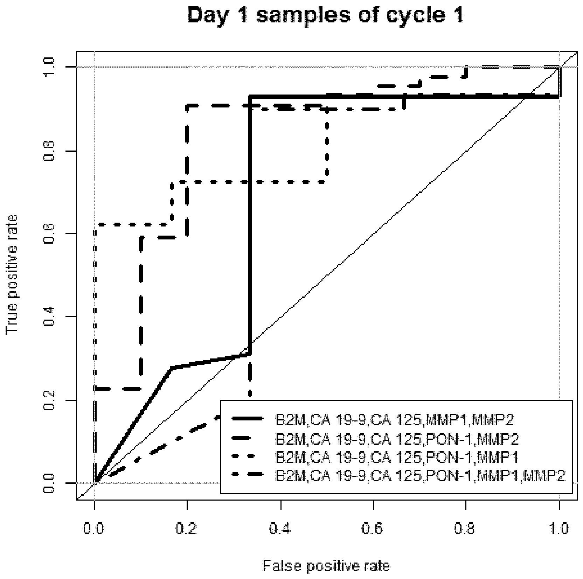

**Figure 2:** Receiver operating characteristics curve (ROC curve) of some diagnostic classifier based on serum levels of preferred combinations of biomarkers (CA-125 protein, CA19-9, β2M protein and PON-1 activity (combination 1); CA-125 protein, CA 19-9, PON-1 activity, MMP-1 protein, and β2M protein (combination 2); or CA-125 protein, CA 19-9, PON-1 activity, MMP-2 protein, and β2M protein (combination 3)). Levels were measured in samples taken at day 1 of menstrual cycle (x-axis: false positive rate, y-axis: true positive rate). A total of 56 (45 with endometriosis vs. 11 without), 37 (31 with endometriosis vs. 6 without) and 54 (44 with endometriosis vs. 10 without) specimens were evaluated for combination 1, 2 and 3, respectively. "Day 1" refers to samples taken on day 1 up to day 4.

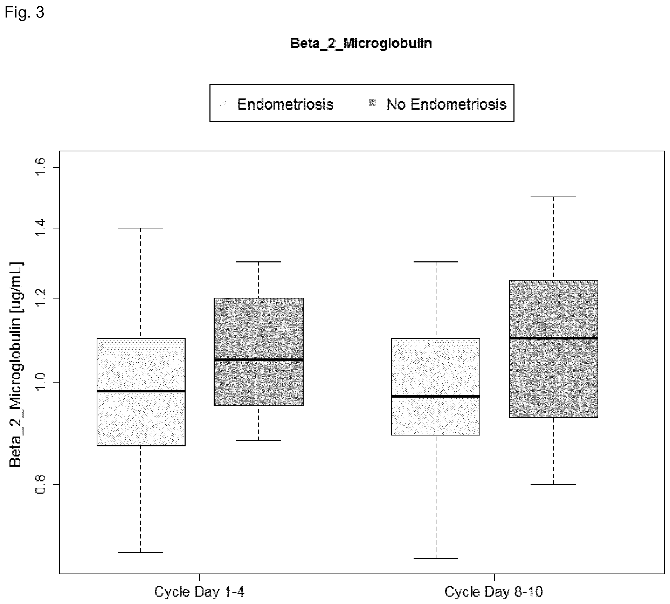

**Figure 3:** Boxplot display the β2M protein level for the subjects with and without endometriosis for the two timepoints namely day 1-4 and day 8-10 of the cycle.

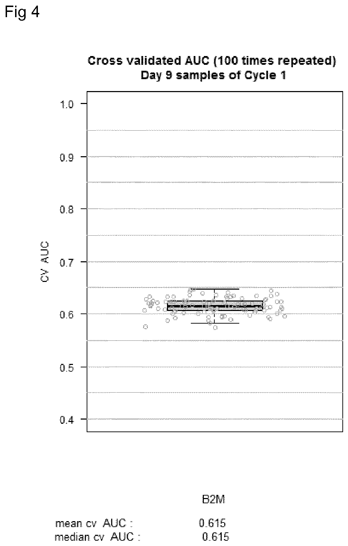

**Figure 4:** Boxplot display of cross validated (cv) area under the curve (AUC) values (y-axis) for β2M protein level

**Detailed Description of the Invention**

In context with the present invention the term *"endometriosis"* relates to the misplaced uterine inner layer tissue (i.e. endometrium). This ectopic endometrium (endometriotic lesions) in endometriotic patients is found outside the uterine cavity, such as in the abdomen on the ovaries, fallopian tubes, and ligaments supporting the uterus; in the area between the vagina and rectum; the outer surface of the uterus; and the peritoneal lining of the pelvic cavity. Other sites for endometriotic lesions may include the bladder, bowel, vagina, cervix, vulva, and in abdominal surgican scars. Further locations of misplaced endometrium include lung, arms, thighs or other places in the body. This misplaced tissue develops into growing lesions and responds to the menstrual cycle, including the menstrual shedding and bleeding. In a particular embodiment the term *"endometriosis"* refers to diseases classified under code N80 of the classification of the World Health Organisation (ICD-10), such as N80.0, N80.1, N80.2, N80.3, N80.4, N80.5, N80.6, N80.7, N80.8, N80.9.

β2-Microglobulin protein refers a component of MHC class I molecules, which are present on all nucleated cells (excludes red blood cells) (see Güssow D, Rein R, Ginjaar I, Hochstenbach F, Seemann G, Kottman A, Ploegh HL (1 November 1987). "The human beta 2-microglobulin gene. Primary structure and definition of the transcriptional unit". J. Immunol. 139 (9): 3132-8). In humans, the β2 microglobulin protein (Cunningham BA, Wang JL, Berggård I, Peterson PA (November 1973). "The complete amino acid sequence of beta 2-microglobulin". Biochemistry. 12 (24): 4811-2) is encoded by the B2M gene. The term β2-Microglobulin protein (or β2M) refers to a protein encoded by the Gene with Gene ID: 567 (see Garth R. et al. (2015), "Gene: a gene-centered information resource at NCBI", Nucleic Acids Res. 2015 Jan 28; 43(Database issue): D36-D42). The levels of β2-Microglobulin protein are preferably determined and used within the method as μg/mL, more preferably as μg/mL in whole blood, more preferably as μg/mL in serum. The skilled person knows how to determine levels of β2-Microglobulin protein in a sample such as serum, e.g. by a immunoassays using a β2-Microglobulin protein-binding antibody or an antigen binding fragment thereof.

CA19-9 (carbohydrate antigen 19-9), is also called cancer antigen 19-9[1] or sialylated Lewis (a) antigen. CA 19-9 is an antigen, a sialylated lacto-N-fucopentose that corresponds to Lewis blood group substance (Magnani, JL (15 June 2004). "The discovery, biology, and drug development of sialyl Lea and sialyl Lex.". Archives of Biochemistry and Biophysics. 426 (2): 122-31). The levels of CA19-9 are preferably determined and used within the method as U/mL, more preferably as U/mL in whole blood, more preferably as U/mL in serum. Units (U) of CA19-9 preferably refers to a gravimetric value of 0.89 ng, i.e. 1 U being 0.89 ng (see Hoon H. Sunwoo and Mavanur R. Suresh, Chapter 9.13 - Cancer Markers, In The Immunoassay Handbook (Fourth Edition), edited by David Wild,, Elsevier, Oxford, 2013, Pages 833-856, ISBN 9780080970370, https://doi.org/10.1016/B978-0-08-097037-0.00067-1).

CA-125 protein (cancer antigen 125, carcinoma antigen 125, or carbohydrate antigen 125) also known as mucin 16 or MUC16 is a protein that is encoded by the MUC16 gene (see Yin BW, Lloyd KO (Jul 2001). "Molecular cloning of the CA125 ovarian cancer antigen: identification as a new mucin, MUC16". The Journal of Biological Chemistry. 276 (29): 27371-5; and Yin BW, Dnistrian A, Lloyd KO (Apr 2002). "Ovarian cancer antigen CA125 is encoded by the MUC16 mucin gene". International Journal of Cancer. Journal International Du Cancer. 98 (5): 737-40). MUC16 is a member of the mucin family glycoproteins (Duraisamy S, Ramasamy S, Kharbanda S, Kufe D (May 2006). "Distinct evolution of the human carcinoma-associated transmembrane mucins, MUC1, MUC4 AND MUC16". Gene. 373: 28-34). The term CA-125 protein refers to a protein encoded by the Gene with Gene ID: 94025 (see Garth R. et al. (2015), "Gene: a gene-centered information resource at NCBI", Nucleic Acids Res. 2015 Jan 28; 43(Database issue): D36-D42). The levels of CA-125 protein are preferably determined and used within the method as U/mL, more preferably as U/mL in whole blood, more preferably as U/mL in serum. Units (U) of CA-125 preferably refers to units as disclosed in Klug, T.L. et al. (1984) Cancer Res. 44:1048.

Paraoxonase-1 (PON-1) is a HDL-associated antioxidant enzyme with paraoxonase, arylesterase and dyazoxonase activities (Gene ID: 5444). PON-1 activity preferably refers to the paraoxonase enzymatic activity of PON-1 within a sample. The skilled person knows how to determine PON-1 activity within a sample. In a preferred embodiment the PON-1 activity is determined using paraoxon (diethyl 4-nitrophenyl phosphate) as the substrate and determining the formation of 4-nitrophenol and change in its concentration after contacting the substrate with the sample suspected to have PON-1activity. Determination of 4-nitrophenol formation and concentration can be conducted by measurement of absorbance change, e.g. at a wave length of 412 nm. There are commercially available kits to determine PON-1 activity [e.g. by Biovision or Thermo Fisher Scientific]. Preferably, "PON-1 activity" refers to its enzymatic activity as determined by paraoxon reaction to 4-nitrophenol as described by Verit et al (see Verit FF, Erel O, Celik N. Serum paraoxonase-1 activity in women with endometriosis and its relationship with the stage of the disease. Hum Reprod. 2008 Jan;23(1): 100-4). Different substrates may be used, e.g. paraoxon or a fluorigenic substrate from Verit et al., 2008: In a preferred embodiment PON-1 activity is determined by using paraoxon as a substrate and measured by increases in the absorbance at 412 nm due to the formation of 4-nitrophenol as already described (Isik et al., 2007). Briefly, the activity may be measured at 25°C by adding 50 ml of the serum to be tested to 1 ml Tris-HCl buffer (100 mM at pH 8.0) containing 2 mM of CaCl2 and 5.5 mM of paraoxon. The rate of generation of 4-nitrophenol is preferably determined at 412 nm. Enzymatic activity is preferably calculated using the molar extinction coefficient 17 100 $M^{-1}$ $cm^{-1}$. The levels of PON-1 activity are preferably determined and used within the method as mmol/min/L, e.g. as determined with above disclosed methods, more preferably as mmol/min/L in whole blood, more preferably as mmol/min/L in serum.

PON-1 activity may be impacted by the presence of one or more polymorphisms in the sequence of PON-1. Polymorphism Q192R (referred to as SNP rs662) is known to be associated with an increase of PON-1 activity. Accordingly, in one embodiment of the invention, the PON-1 activity is determined by determining the presence of an activity enhancing variation in PON-1 gene or protein. Further, in a preferred embodiment the PON-1 activity is determined by determining the presence of an activity enhancing SNP (single nucleotide polymorphism) in PON-1 gene. Preferably, the PON-1 activity is determined by determining the presence of an activity enhancing SNP altering the PON-1 protein (UniProtKB - P27169; SEQ ID NO:1) at the amino acid at position 192. In one particular embodiment, PON-1 activity is determined the presence of SNP rs662(R), wherein the presence of SNP rs662(R) is attributed to an increased PON-1 activity. SNP rs662(R) is known to the person skilled in the art and preferably refers to a polymorphism resulting in an exchange of the amino acid Q at position 192 by the amino acid R.

It is known by the skilled person that a linkage disequilibrium of the missense mutation rs622 with the intronic SNPs rs2237582, rs2269829, rs2057681, rs3917534, rs3917532, rs3917529, and rs1157745 (Source: HaploReg v4.1, http://archive.broadinstitute.org/mammals/haploreg/haploreg.php) Hence, in one embodiment the status of SNP rs622 is determined by determining the status of one or more SNP selected from the group consisting of rs2237582, rs2269829, rs2057681, rs3917534, rs3917532, rs3917529, and rs1157745.

Matrix metalloproteinase-1 protein (MMP-1 protein or MMP1 protein) also known as interstitial collagenase and fibroblast collagenase is an enzyme that in humans is encoded by the MMP1 gene (see Brinckerhoff CE, et al. (February 1987). "Molecular cloning of human synovial cell collagenase and selection of a single gene from genomic DNA". J. Clin. Invest. 79 (2): 542-6; and .Pendas AM, et al. (October 1996). "Fine physical mapping of the human matrix metalloproteinase genes clustered on chromosome 11 q22.3". Genomics. 37 (2): 266-8). The term MMP1 protein refers to a protein encoded by the Gene with Gene ID: 4312 (see Garth R. et al. (2015), "Gene: a gene-centered information resource at NCBI", Nucleic Acids Res. 2015 Jan 28; 43(Database issue): D36-D42). The levels of MMP-1 protein are preferably determined and used within the method as ng/mL, more preferably as ng/mL in whole blood, more preferably as ng/mL in serum.

Matrix metalloproteinase-2 protein (MMP-2 protein or MMP2 protein) is also known as 72 kDa type IV collagenase and gelatinase A. MMP2 protein is an enzyme that in humans is encoded by the MMP2 gene (see Devarajan P, et al. (December 1992). "Structure and expression of neutrophil gelatinase cDNA. Identity with type IV collagenase from HT1080 cells". J. Biol. Chem. 267 (35): 25228-32). The term MMP2 protein refers to a protein encoded by the Gene with Gene ID: 4313 (see Garth R. et al. (2015), "Gene: a gene-centered information resource at NCBI", Nucleic Acids Res. 2015 Jan 28; 43(Database issue): D36-D42). The levels of MMP-2 protein are preferably determined and used within the method as ng/mL, more preferably as ng/mL in whole blood, more preferably as ng/mL in serum.

The term *"control lever"* refers to the respective biomarker level found in the respective samples of one or more subjects not having endometriosis. In a preferred embodiment, "control level" refers to the respective biomarker level found in the respective samples of one or more subjects having endometriosis symptoms but not having endometriosis. Further preferred, "control level" refers to the respective biomarker level found in the respective samples of one or more subjects suffering from chronic pelvic pain, dysmenorrhea, dyspareunia and/or infertility but not having endometriosis. The skilled person will also understand that the "control level" may be implicated in the used assay for detecting said biomarker. The skilled person hence may chose particulars of the assay so that the test is positive the indication of endometriosis for the respective biomarker in the sample if levels above or below a certain level are reached and *vice versa* is negative for the indication of endometriosis if levels are determined that are below or above the control level, depending on the respective biomarker. As further outlined below, control levels may be superfluous if the tested biomarker is part of a diagnostic classifier. In such case, a classification method like for example a logistic regression model may be used to relate the determined levels of the biomarkers to a diagnostic output. In logistic regression models control levels are substituted by the factors of the algorithm. Other classification methods including, but not limited to penalized regression models, decision trees and support vector machines may also be used.

For $\beta$2-M protein level as a biomarker, the control level below which the biomarker is regarded as indicative for the presence of endometriosis in the subject to be diagnosed is preferably 1.3 $\mu$g/mL or less, preferably 1.1 $\mu$g/mL or less. Depended on the desired specificity and/or specificity of the diagnostic method different control levels may be chosen. Preferred control levels for $\beta$2-M protein level are 1.3 $\mu$g/mL or less, 1.1. $\mu$g/mL or less, 1.0 $\mu$g/mL or less, or 0.88 $\mu$g/mL or less. As outlined herein, the actual control level may be dependent on the day of the menstrual cycle the sample has been taken. Preferred control levels for $\beta$2-M protein level as a biomarker in samples taken on days 1 to 4 of the menstrual cycle of the subject to be diagnosed are selected from the group consisting of 1.1 $\mu$g/mL or less, 1.0 $\mu$g/mL or less, and 0.88 $\mu$g/mL or less. Preferred control levels for $\beta$2-Microglobulin protein level as a biomarker in samples taken on days 8 to 10 of the menstrual cycle of the subject to be diagnosed are selected from the group consisting of 1.8 $\mu$g/mL or less, 1.0 $\mu$g/mL or less, and 0.88 $\mu$g/mL or less.

*"Diagnosis"* in context with the present invention preferably denotes the assessment of a disease status of a subject. In one embodiment it also refers to risk stratification, prognosis, monitoring, or therapy control. In the present invention, the term risk stratification denotes a statistical process by which the quality of a certain form of treatment can be assessed independently of patient case-mix. Preferably diagnosis refers to diagnosis. Thus, the quality of certain form of treatment for a given medical condition may generally be assessed independently of certain risk factors which influence the outcome of said treatment, such as for example, the age, ethnic background, genetic predispositions, prior history of diseases and the like. Equally, the assessment may be made for certain sub-groups of subjects, e.g. of a certain age, in order to assess the quality of a certain form of treatment for said sub-group. In the present invention, the term prognosis denotes a prediction of how a subject's (e.g. a patient's) medical condition will progress. This may include an estimation of the chance of recovery or the chance of an adverse outcome for said subject. In the present invention, the term monitoring denotes the observation of the state or progression of a subject's medical condition by measuring the level of a certain diagnostic marker or markers for said medical condition at various points of time. In the present invention, the term therapy control denotes the attribution of a certain form of treatment, such as the administration of a medicament, to the state or progression of a subject's medical condition by measuring the level of a certain diagnostic marker or markers for said medical condition at various points of time, preferably before and after the treatment. In this way, it may be determined whether said treatment is adequate to treat said medical condition, or whether the therapy will have to be adjusted, e.g. by altering the dosage of the medicament, or will have to be replaced by another form of treatment, e.g. another medicament.

The term *"subject to be diagnosed"* or *"patient"* refers to a female mammal, preferably a female human. Preferably the subject to be diagnosed is suspected to suffer of endometriosis, e.g. a female human suspected to suffer of endometriosis. The subject to be diagnosed may be not being diagnosed before. However, in one embodiment the subject suspected to suffer of endometriosis has received an initial diagnosis or shows symptoms of endometriosis. Hence, in one embodiment the subject to be diagnosed suffers from one or more symptoms selected from the group consisting of chronic pelvic pain, dysmenorrhea, dyspareunia and infertility. In a particular preferred embodiment the subject to be diagnosed suffers from chronic pelvic pain. Further preferred the subject to be diagnosed is a female human suffering from chronic pelvic pain and suspected to suffer from endometriosis.

*"Infertility"* (also referred to as "subfertility") in connection with the present invention preferably relates to any form of reduced fertility with prolonged time of unwanted non-conception, preferably the failure to establish a clinical pregnancy after 12 months of regular and unprotected sexual intercourse (see Vander Borght M & Wyns C. Fertility and infertility: Definition and epidemiology, Clin Biochem. 2018 Mar 16).

A *"biomarker"* or *"marker"* in the context of the present invention refers to the level of an organic biomolecule, particularly a polypeptide, or its activity, as well as carbohydrate as disclosed herein, which is differentially present in a sample taken from subjects having a certain condition as compared to a comparable sample taken from one or more subjects who do not have said condition. For examples, a marker can be a polypeptide or its enzymatic activity or carbohydrate which is present at an elevated or decreased level in samples of endometriosis patients compared to samples of patients with a negative diagnosis.

The method of the present invention is preferably performed in a sample. It is hence, in a preferred embodiment an *in vitro* or *ex vivo* method. The method may comprise the step of *"providing a sample of a subject to be diagnosed"*. The skilled person will instantly understand that the sample is to be provided before the determination of the respective biomarker in the sample is performed.

The inventors have further found that the method for diagnosis of endometriosis can be further enhanced if CA19-9 level is included as a further biomarker. The inventors found that increased levels of CA19-9 are indicative of endometriosis.

Hence, in an embodiment of the present invention, the method for diagnosis of endometriosis in a subject further comprises the steps of

(iii) determining the level of CA19-9 as a second biomarker in said sample of said subject to be diagnosed,
(iv) comparing the second biomarker level as determined in steps (iii) to a respective control level derived from one or more subject without endometriosis;

wherein an increased level as compared to the respective control level of CA 19-9 is indicative for the presence of endometriosis in said subject to be diagnosed.

Accordingly, the method may comprise the determination of two or more biomarkers, wherein the two or more biomarker includes levels of β2M protein and CA19-9. The determination of the biomarker levels may take place in parallel or subsequent. The skilled person will instantly understand that steps (i) and (iii) may be merged and that also steps (ii) and (iv) of the inventive method for diagnosis may be performed in parallel. Hence, the present invention also relates to a method for diagnosis of endometriosis in a subject, the method comprising the steps of

(i) determining the level of β2-Microglobulin protein (β2M protein) as a first biomarker and the level of CA19-9 as a second biomarker in a sample of said subject to be diagnosed;
(ii) comparing the biomarker levels as determined in steps (i) to a respective control levels derived from one or more subject without endometriosis;

wherein a reduced level as compared to the respective control level of β2M protein is indicative for the presence of endometriosis in said subject to be diagnosed, and
wherein an increased level as compared to the respective control level of CA 19-9 is indicative for the presence of endometriosis in said subject to be diagnosed.

The inventors further found that if one or more further biomarkers are added to the first biomarker in addition to CA19-9, the diagnostic value of the method may be yet further enhanced. Hence, in an embodiment of the method according to the present invention in addition to the second and the first biomarker the level of one or more further biomarker selected from the group consisting of CA125 protein, PON-1 activity, MMP1 protein and MMP2 protein is determined and compared to a respective control level derived from one or more subject without endometriosis.

For any particular biomarker or combination of biomarkers, a distribution of biomarker levels for subjects with and without a disease will likely overlap. Under such conditions, a test or classifier does not absolutely distinguish normal from disease with 100% accuracy, and the area of overlap indicates where the test cannot distinguish normal from disease. A threshold is selected, below which the test is considered to be abnormal and above which the test is considered to be normal. The ability of the test to distinguish between normal and disease is evaluated by means of a Receiver Operating Characteristic curve (ROC curve), which summarizes the sensitivities and specificities of the test or classifier for all possible thresholds.. In addition to accuracy, sensitivity and specificity the area under the ROC curve (AUC) is a value to describe the performance of the classifier but in contrast to the other three independent of the cut off. The AUC can have values between 0 and 1. In practice, ROC curves are typically generated by calculation the relative frequencies in terms of sensitivity (true positive rate) and specificity (true negative rate) for each value of the marker or combination of markers. Sensitivity will be plotted on the vertical axis and 1- specificity (false positive rate) on the horizontal axis. True positive rate is the rate of subjects having laparoscopy-confirmed endometriosis within the group of subjects classified as having endometriosis using the test or classifier. False positive rate is the rate of subjects showing no endometriosis in diagnostic laparoscopy within the group of subjects classified as having endometriosis using the test or classifier. True negative rate is the rate of subjects showing no endometriosis in diagnostic laparoscopy within the subjects classified as normal (no-endometriosis) using the test or classifier. Thus, for a particular cut-off selected, the value of (1-specificity) may be determined, and a corresponding sensitivity may be obtained. The area under the receiver operating characteristic curve (often referred to as simply the AUC) is equal to the probability that a classifier will rank a randomly chosen positive instance higher than a randomly chosen negative one (assuming 'positive' ranks higher than 'negative'). Thus, the area under the ROC curve can be used to determine the effectiveness of the test, independent of a cut-off or control level. Preferably, an area under the ROC curve of about 0.6 or more is indicative for the effectiveness of the classifier to distinguish between subjects to be diagnosed with and without a disease, preferably of about 0.65 or more, more preferred of about 0.7 or more, yet more preferred of about 0.8 or more, even more preferred of about 0.9 or more.

The term "about" in context with the present invention refers to+/- 5% of a given measurement. The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test. In one embodiment the biomarkers of the method according to the present inventions are comprised in a diagnostic classifier.

The term *"diagnostic classifier"* refers to a biomarker or a plurality of biomarkers (panel) that allow a calculation of a classification, score or probability for a certain disease based on a mathematical algorithm. Such algorithm is modulated based on analytical data of the respective biomarkers from samples derived from diseased and non-diseased patients.

Within such diagnostic classifier the necessity for control levels and the respective comparison may be overcome. Further, the skilled person will understand that the control levels may be exchanged by calculating the probability for the presence of the disease within a diagnostic classifier. In brief, using diagnostic classifier comprises a plurality of biomarkers, the levels of which are determined. Based on the determined levels of the biomarkers the comparison of the levels to control levels may be performed by calculating the probability for endometriosis. The skilled person will acknowledge that the control levels may not necessary be determined within the diagnostic methods, but may form part of a logistic regression model in which the different biomarkers are differently weighted within the classifier through the use of different weighing factors.

Hence, in one embodiment the comparison of the levels of said biomarkers of the diagnostic classifier with the respective control levels is performed by calculating the probability for endometriosis using a logistic regression model.

In summary, probabilities for a diagnosis may be calculated by the skilled person based on the present invention using suited models. Preferred models are disclosed herein.

*"Logistic model",* or *"logistic regression model"(used* interchangeably herein) refer to a regression model where the dependent variable is categorical, such as binary. A logistic regression model is a statistical model to describe the probability of a binary or categorical depended variable (diagnosis of endometrioses yes/no) by one or more independent variables (biomarkers). The model parameters are calculated in a way that the model, given as a mathematical function describes the depended variable optimal using the independent variables, based on the given data. The result of the model application of a logistic regression model is the probability for the diagnosis of endometriosis. Logistic regression models are nonlinear to reflect the sigmoidal shape of the probability function. The probability of endometriosis may be classified into a diagnosis of endometriosis. Iff the probability for a given subject is above a certain level, the subject will be classified as having the disease. A probability has levels between 0 and 1, meaning between 0 and 100%.

In a preferred embodiment a probability of more than 0.6 is indicative for the presence of endometriosis in said subject to be diagnosed, preferably of 0.65 or more, more preferred of 0.7 or more, yet more preferred 0.8 or more, even more preferred 0.9 or more.

In a preferred embodiment of the invention, the calculation of probability for endometriosis is performed with a logistic regression model, also based on the determined biomarker levels. In particular, the probability for having endometriosis is calculated based on a weighted sum of the determined biomarker levels in the nonlinear logistic regression model More particular, the probability for the presence of endometriosis (in the following referred to as $P(Y = 1|x)$) is calculated with the logistic model based on the determined biomarker levels x by

$$P(Y = 1|x) = \frac{1}{\left(1 + \exp(-b_0 - x^T b)\right)};$$

wherein $b$ is the vector of weighing factors ($b = (b_1, \ldots, b_p)^T$) for the determined biomarkers;
wherein $x$ is the vector of the determined levels for the respective biomarkers($x = (x_1, \ldots, x_p)^T$),
wherein $p$ is the number of determined biomarkers within the combination; and
wherein $b_0$ is the intercept in the model for the specific combination of biomarkers.
The weighing factors $b$ and the additional model parameter $b_0$ are calculated based on the given data set, so that the relation between the presence of endometriosis and the measured biomarker levels is optimized in terms of the given model. In the following tables $b_1, \ldots, b_p$ are referred to as weighting factors for the respective biomarkers within the combination, whereas $b_0$ refers to an additional model parameter (also referred to as intercept) of the combination.
Terminology used herein in connection with the above model is known by those skilled in the art of mathematics and statistics; e.g. the skilled person will recognize that "T" refers to the term "transposed" in its mathematical sense.
In an embodiment of the present invention, the diagnostic classifier comprises β2M protein levels and CA19-9 levels as biomarkers. However, as outlined herein, further biomarkers may be added. Hence, in one embodiment the diagnostic classifier comprises 3 or more biomarkers, preferably 4 or more, yet more preferred 5 or more. As outlined above, the further biomarkers are preferably selected from the group of further biomarkers consisting of CA125 protein level, PON-1 activity, MMP1 protein level and MMP2 protein level. In a preferred embodiment the diagnostic classifier, hence, comprises 3 or more biomarkers selected from the group consisting of β2M protein level, CA 19-9 level, CA125 protein level, PON-1 activity, MMP1 protein level and MMP2 protein level. In other words, the diagnostic classifier comprises β2M protein level, CA 19-9 level, and at least one further biomarker selected from the group consisting of CA125 protein level, PON-1 activity, MMP1 protein level and MMP2 protein level. In a further preferred embodiment the diagnostic classifier comprises 4 or more biomarkers selected from the group consisting of β2M protein level, CA 19-9 level, CA125 protein level, PON-1 activity, MMP1 protein level and MMP2 protein level. In other words, the diagnostic classifier comprises β2M protein level, CA 19-9 level, and at least two further biomarkers selected from the group consisting of CA125 protein level, PON-1 activity, MMP1 protein level and MMP2 protein level. In a further preferred embodiment the diagnostic classifier comprises 5 or more biomarkers selected from the group consisting of β2M protein level, CA 19-9 level, CA125 protein level, PON-1 activity, MMP1 protein level and MMP2 protein level. In other words, the diagnostic classifier comprises β2M protein level, CA 19-9 level, and at least three further biomarker selected from the group consisting of CA125 protein level, PON-1 activity, MMP1 protein level and MMP2 protein level.
As will be readily understood by the skilled person, the present invention provides for a group of novel and superior biomarkers that allow diagnosis of endometriosis. The skilled person is in the position with the herein provided knowledge of specific biomarkers to determine suited combinations within these biomarkers for the method according to the invention. However, the inventors have exemplified their invention by showing the diagnostic value of the biomarkers according to the invention by using twelve different combinations (combinations 1 to 12) with different numbers of biomarkers within the classifier. Hence, in certain aspects of the present invention the diagnostic classifier comprises a combination of biomarker selected from the group combinations consisting of

β 2M protein level and CA 19-9 level (combination 1)
β 2M protein level, CA-125 protein level and CA 19-9 level (combination 2)
β 2M protein level, PON-1 activity and CA 19-9 level (combination 3)
β 2M protein level, CA 19-9 level and MMP1 protein level (combination 4)
β 2M protein level, CA 19-9 level, PON-1 activity and MMP2 protein level (combination 5)
β 2M protein level, CA-125 protein level, CA 19-9 level and MMP1 protein level (combination 6)
β 2M protein level, CA-125 protein level, CA 19-9 level and MMP2 protein level (combination 7)
β 2M protein level, CA-125 protein level, CA 19-9 level and PON-1 activity (combination 8)
β 2M protein level, CA-125 protein level, CA 19-9 level, MMP1 protein level and MMP2 protein level (combination 9)
β 2M protein level, CA-125 protein level,CA 19-9 level, PON-1 activity and MMP2 protein level (combination 10)
β 2M protein level, CA-125 protein level,CA 19-9 level, PON-1 activity and MMP1 protein level (combination 11)
β 2M protein level, CA-125 protein level,CA 19-9 level, PON-1 activity, MMP1 protein level and MMP2 protein level (combination 12).
The preferred combinations within the diagnostic classifier are preferably subjected to a logistic regression model as described herein. Depending on the actual combination comprised in the diagnostic classifier, a respective biomarker within the classifier may contribute to increased probability if present in higher or lower levels. This is reflected by the algebraic sign before the weighing factor ($b_1$ to $b_p$). In a preferred embodiment, the algebraic signs before the weighing factors in the logistic regression model according to the invention for the respective biomarkers in the specific combinations are as follows:

Table 1:

| Combination | β2M protein level | CA 19-9 level | CA-125 protein level | PON-1 activity | MMP1 protein level | MMP2 protein level |
|---|---|---|---|---|---|---|
| 1 | - | + | n/a | n/a | n/a | n/a |
| 2 | - | + | + | n/a | n/a | n/a |
| 3 | - | + | n/a | + | n/a | n/a |
| 4 | - | + | n/a | n/a | + | n/a |
| 5 | - | + | n/a | + | n/a | - |
| 6 | - | + | - | n/a | + | n/a |
| 7 | - | + | + | n/a | n/a | - |
| 8 | - | + | + | + | n/a | n/a |
| 9 | - | + | - | n/a | + | - |
| 10 | - | + | + | + | n/a | - |
| 11 | - | + | - | - | + | n/a |
| 12 | - | + | - | - | + | - |

Further, the weighing factor within the logistic regression model reflects the scale with which the determined level of the respective biomarker contributes to the probability of endometriosis. In a preferred embodiment the biomarkers within the respective combination have model parameters (weighing factors and intercept) according to the following:

Table 2:

Combination 1:
Intercept ($b_0$): 2.8

| Biomarker | Weighing factor ($b_1$ to $b_p$) |
|---|---|
| CA19-9 | 0.21 |
| β2M protein | -3.0 |

Combination 2:
Intercept ($b_0$): 3.9

| Biomarker | Weighing factor ($b_1$ to $b_p$) |
|---|---|
| CA19-9 | 0.33 |
| β2M protein | -4.6 |
| CA-125 protein | 0.015 |

Combination 3:
Intercept ($b_0$): 2.0

| Biomarker | Weighing factor ($b_1$ to $b_p$) |
|---|---|
| PON1 activity | 0.0047 |
| CA19-9 | 0.20 |
| β2M protein | -3.2 |

(continued)

Combination 3:

Intercept ($b_0$): 2.0

| Biomarker | Weighing factor ($b_1$ *to* $b_p$) |
| --- | --- |

Combination 4:

Intercept ($b_0$): 6.5

| Biomarker | Weighing factor ($b_1$ *to* $b_p$) |
| --- | --- |
| MMP1 protein | 0.18 |
| CA19-9 | 0.36 |
| β2M protein | -7.8 |

Combination 5:

Intercept ($b_0$): 3.8

| Biomarker | Weighing factor ($b_1$ *to* $b_p$) |
| --- | --- |
| MMP2 protein | -0.00089 |
| PON1 activity | 0.0050 |
| CA19-9 | 0.22 |
| β2M protein | -3.7 |

Combination 6:

Intercept ($b_0$): 15

| Biomarker | Weighing factor ($b_1$ *to* $b_p$) |
| --- | --- |
| MMP1 protein | 0.66 |

| Biomarker | Weighing factor ($b_1$ *to* $b_p$) |
| --- | --- |
| CA19-9 | 1.1 |
| β2M protein | -19 |
| CA-125 protein | -0.012 |
| (Intercept) | 15 |

Combination 7:

Intercept ($b_0$): 6.3

| Biomarker | Weighing factor ($b_1$ *to* $b_p$) |
| --- | --- |
| MMP2 protein | -0.00077 |
| CA19-9 | 0.38 |
| β2M protein | -5.8 |
| CA-125 protein | 0.013 |

Combination 8:

Intercept ($b_0$): 2.87

| Biomarker | Weighing factor ($b_1$ *to* $b_p$) |
| --- | --- |
| PON1 activity | 0.0093 |
| CA19-9 | 0.335 |
| β2M protein | -5.28 |
| CA-125 protein | 0.0166 |

(continued)

| Combination 9: | |
| Intercept ($b_0$): 16 | |
| **Biomarker** | **Weighing factor ($b_0$ to $b_p$)** |
| MMP2 protein | -0.0010 |
| MMP1 protein | 0.33 |
| CA19-9 | 1.2 |
| β2M protein | -18 |
| CA-125 protein | -0.023 |

| Combination 10: | |
| Intercept ($b_0$): 5.4 | |
| **Biomarker** | **Weighing factor ($b_1$ to $b_p$)** |
| MMP2 protein | -0.0011 |
| PON1 activity | 0.011 |
| CA19-9 | 0.42 |
| β2M protein | -6.4 |
| CA-125 protein | 0.011 |

| Combination 11: | |
| Intercept ($b_0$): 21 | |
| **Biomarker** | **Weighing factor ($b_1$ to $b_p$)** |
| MMP1 protein | 0.61 |
| PON1 activity | -0.0055 |
| CA19-9 | 1.4 |
| β2M protein | -25 |
| CA-125 protein | -0.017 |
| Combination 12: | |
| Intercept ($b_0$): 20 | |
| **Biomarker** | **Weighing factor ($b_1$ to $b_p$)** |
| MMP2 protein | -0.00095 |
| MMP1 protein | 0.35 |
| PON1 activity | -0.0031 |
| CA19-9 | 1.3 |
| β2M protein | -21 |
| CA-125 protein | -0.019 |

In context of the logistic regression model with the specific weighing factors and intercepts as disclosed herein the respective levels of the biomarkers are given with the numerical value as determined in the following units:

the levels of β2-Microglobulin protein are preferably determined and used within the method as μg/mL, more preferably as μg/mL in whole blood, more preferably as μg/mL in serum;
the levels of CA19-9 are preferably determined and used within the method as U/mL, more preferably as U/mL in whole blood, more preferably as U/mL in serum;
the levels of CA-125 are preferably determined and used within the method as U/mL, more preferably as U/mL in whole blood, more preferably as U/mL in serum;
the levels of PON-1 activity are preferably determined and used within the method as mmol/min/L, more preferably as mmol/min/L in whole blood, more preferably as mmol/min/L in serum;
the levels of MMP-1 are preferably determined and used within the method as ng/mL, more preferably as ng/mL in whole blood, more preferably as ng/mL in serum; and

the levels of MMP-2 are preferably determined and used within the method as ng/mL, more preferably as ng/mL in whole blood, more preferably as ng/mL in serum.

By "numerical value" it is meant that the determined level is used within the algorithm without the actual unit of the determined level.

As endometriotic tissue is influenced by the menstrual cycle of the patient, it may be preferred to take the sample within a certain time frame of the menstrual cycle. In context with the present invention, the start of the menstrual cycle is the day of onset of menstrual bleedings. The day preferred may be dependent on the actual classifier or biomarker used. In a preferred embodiment of the present invention, the sample is collected from said subject to be diagnosed within the first ten days of the menstrual cycle, preferably within the first week. A preferred day for diagnostic classifiers comprising a combination of biomarkers as disclosed herein is within day 1 to 4 of the menstrual cycle. For $\beta$2-Microglobulin protein level as a single marker a sample collected on any of day 8 to 10 is preferred.

Levels of biomarkers in samples as such may be detected by any method suitable for specifically detecting the presence or level of a biomarker. Such methods are known by the person skilled and include immunoassays that come in many different formats and variations.

"Immunoassays" in the meaning of the invention are assays utilizing the specific interaction between the target biomarker or antigenic fragments thereof and an antibody specifically binding said biomarker or antigenic fragments thereof, in order to detect the presence or determine the concentration of the target biomarker. For example, the detection and quantification of a biomarker in a sample can be performed with the aid of said antibodies targeting the biomarker of interest or an antigen binding fragments thereof, e.g. by immunoprecipitation or immunoblotting. For example, immunoassays in the meaning of the invention can be subdivided into the following steps: (1) biomarker/biomarker-antibody reaction, (2) if required separation of the biomarker/biomarker-antibody complex from other components of the reaction mixture especially from non-bound biomarker and non-bound biomarker-antibody and (3) measuring the response. As for the biomarker/biomarker-antibody reaction various configurations are passable, e.g. (a) precipitation of one reaction with an excess of the other or (b) competition between known quantities of biomarker and biomarker-antibody and the material to be investigated. Immunoassays are known in the art and refer to an assay in which a certain analyte is detected using specific antibody antigen interaction, i.e. the binding of an antibody to its antigen. Immunoassays may be run in multiple steps with reagents being added and washed away or separated at different points in the assay. Multi-step assays are often called separation immunoassays or heterogeneous immunoassays. Some immunoassays can be carried out simply by mixing the reagents and sample and making a physical measurement. Such assays are called homogenous immunoassays or less frequently non-separation immunoassays. In a preferred embodiment the levels of the biomarker proteins are detected in an Immunoassay. The skilled person will acknowledge that the different protein level biomarkers may be detected in parallel or in a single assay. Suitable single assays include protein microchip assays. In one embodiment the immunoassay is selected from the group of immunoprecipitation, enzyme immunoassay (EIA), radioimmunoassay (RIA), enzyme-linked immunosorbent assay (ELISA), fluorescent Immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay.

The immunoassays can be homogenous or heterogeneous assays, competitive and noncompetitive assays. In a particularly preferred embodiment, the assay is in the form of a sandwich assay, which is a non-competitive immunoassay, wherein the biomarker protein (i.e. the "analyte") to be detected and/or quantified is allowed to bind to an immobilized biomarker protein specific antibody (primary antibody) or antigen binding fragment thereof and to a secondary antibody or antigen binding fragment thereof. The primary antibody or antigen binding fragment thereof, may e.g., be bound to a solid phase, e.g. a bead, a surface of a well or other container, a chip or a strip, and the secondary antibody is an antibody which is labeled, e.g. with a dye, with a radioisotope, or a reactive or catalytically active moiety such as a peroxidase, e.g. horseradish peroxidase. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267; Hultschig C et al., Curr Opin Chern Bioi. 2006 Feb;l0(1):4-10. PMID: 16376134, incorporated herein by reference). Sandwich immunoassays can for example be designed as one-step assays or as twostep assays.

As the detection of $\beta$2M and the other biomarkers is useful for the diagnostic method of the invention, the invention also relates to the use of $\beta$2-Microglobulin ($\beta$2M) binding antibody or an antigen binding peptide fragment thereof, and one or more antibodies or an antigen binding peptide fragment thereof binding one of more further biomarkers selected from the group consisting of CA125, CA19-9, PON-1 activity, MMP1, and MMP2 for the diagnosis of endometriosis or monitoring the response of a subject to the treatment for endometriosis. In a preferred embodiment the use comprises the use of two or more antibodies or antibody binding peptide fragments thereof binding two or more of said further biomarkers.

The term *"antibody"* comprises monoclonal and polyclonal antibodies and antigen binding fragments thereof, in particular Fc-fragments as well as so called "single-chain-antibodies" (Bird R. E. et al (1988) Science 242:423-6), chimeric, humanized, in particular CDR-grafted antibodies, and dia- or tetrabodies (Holliger P. et al (1993) Proc. Natl. Acad. Sci. U.S.A. 90:6444-8). Also comprised are immunoglobulins like proteins that are selected through techniques including, for example, phage display to specifically bind to the polypeptides of the protein level biomarkers of the present invention. In this context the term "specific binding" refers to antibodies raised against peptides derived from the protein level biomarkers. Such peptides can comprise additional or less N- or C-terminal amino acids. The antibodies of the invention specifically bind to the respective biomarker protein molecule or an antigenic fragment thereof. Binding occurs through binding of the epitope on the protein or fragment by the antibody or at least the epitope binding fragment thereof. The antibody can also be modified (e.g. oligomeric, reduced, oxidized and labeled antibodies). The term antibody as used herein comprises both intact molecules and also antigen-binding antibody fragments such as Fab, F(ab')z and Fv capable of binding specific epitope determinants of the biomarker protein. In these fragments the antibody(ies) capability of selectively binding its antigen or receptor is retained in part, the fragments being defined as follows: (1) Fab, the fragment which contains a monovalent antigen-binding fragment of an antibody molecule, can be generated by cleavage of a whole antibody using the enzyme papaine, thereby obtaining an intact light chain and part of a heavy chain; (2) the Fab fragment of an antibody molecule can be produced by treatment of a whole antibody with pepsin and subsequent reduction, thereby obtaining an intact light chain and part of a heavy chain, two Fab fragments per antibody molecule are obtained; (3) F(ab')2 the fragment of the antibody which can be obtained by treatment of a whole antibody with the enzyme pepsin without subsequent reduction, F(ab')2 is a dimer comprised of two Fab fragments held together by two disulfate bonds; (4) Fv defined as fragment modified by genetic engineering which includes the variable region of the light chain and the variable region of the heavy chain is expressed in the form of two chains; and (5) single-chain antibody (SCA) defined as a molecule modified by genetic engineering, which includes the variable region of the light chain and the variable region of the heavy chain, linked by a suitable polypeptide linker to perform a genetically fused single-chain molecule. Also included in the term antibody(ies) are diabodies, single-domain antibody(ies) (sdAb, also referred to as Nanobody(ies)).

The term "epitope" as used in the present invention represents any antigen determinant on the biomarker protein of interest. Epitope determinance normally consists of chemically active surface groups of molecules such as amino acids or sugar-side chains and normally has specific features of the free dimensional structure as well as specific chart properties. The antibody binds specifically to the protein or in doing so shows specific immune-reactivity when the antibody assumes its function in a binding reaction in the presence of a heterogeneous population of proteins or fragments thereof, thereby allowing a conclusion whether the protein or another biological structure is present. Under the present conditions of an immunoassay, the above-mentioned antibodies will preferably bind to a specific portion of the respective biomarker protein, while no significant binding to other proteins present in the sample will take place.

**Disclosed Sequences:**

SEQ ID NO: 1 (PON-1 protein sequence)

```
MAKLIALTLL   GMGLALFRNH   QSSYQTRLNA   LREVQPVELP   NCNLVKGIET   GSEDLEILPN

GLAFISSGLK   YPGIKSFNPN   SPGKILLMDL   NEEDPTVLEL   GITGSKFDVS   SFNPHGISTF

TDEDNAMYLL   VVNHPDAKST   VELFKFQEEE   KSLLHLKTIR   HKLLPNLNDI   VAVGPEHFYG

TNDHYFLDPY   LQSWEMYLGL   AWSYVVYYSP   SEVRVVAEGF   DFANGINISP   DGKYVYIAEL

LAHKIHVYEK   HANWTLTPLK   SLDFNTLVDN   ISVDPETGDL   WVGCHPNGMK   IFFYDSENPP

ASEVLRIQNI  LTEEPKVTQV  YAENGTVLQG  STVASVYKGK  LLIGTVFHKA  LYCEL
```

**EXAMPLES**

**Material & Methods**

**Clinical study design & serum collection**

A prospective biomarker research study was conducted in multiple centers in Germany. Within this study, serum samples for diagnostic biomarkers were collected.

Serum was obtained from 124 women (age 18-45 years) undergoing laparoscopy due to endometriosis associated pain symptoms (chronic pelvic pain), infertility, and/or tubal ligation. Women were observed for 3 full menstrual cycles, defined as menstrual cycles with a luteinizing hormone (LH) surge, detected by ClearPlan Fertility Monitor (Procter & Gamble, Schwalbach, Germany). During the first cycle, venous blood samples were collected preovulatory on day 1 (up to day 4) or day 9 (+/- 1d) of the menstrual cycle and on days 3 and 11 after LH surge (LH + 3d and LH + 11 d). During the proliferative phase (days 4 to 11) of the second cycle, a diagnostic laparoscopy was carried out. Among the subgroup of 97 women with pain symptoms suggestive of endometriosis, disease presence was confirmed through laparoscopy and histopathology in 69 women, whereas 28 symptomatic women showed no evidence of endometriotic lesions but having chronic pain. The subgroup of 27 women with no typical symptoms of endometriosis (i.e., no endometriosis-associated pain) revealed 11 women with endometriotic lesions and 16 without. Symptomatic women with no laparoscopic evidence of endometriosis served as the control group for symptomatic endometriosis patients.

**Assessment of biomarkers in serum**

The biomarkers described were investigated by immunolabelling techniques for biomarker levels (CA-125 protein, CA19-9, MMP1 protein, MMP2 protein, $\beta$2M protein) or in an activity assay in case of PON-1.

In detail, CA-125 protein level was measured by utilizing Immulite assay technology (Supplier# OM-MA 005333, Ref L2KOP2]), which is a two-site sandwich immunochemiluminometric assay using murine monoclonal capture and detection antibodies with an analytical measurement range of 1 to 5,000 U/mL. CA19-9 level and $\beta$2M protein level were measured utilizing the Luminex multiplex platform (utilizing polystyrene microspheres, Myriad RBM), while analysis of MMP1 protein and MMP2 protein levels employed commercially available Biotrak™ sandwich-ELISA assays (both from GE Healthcare, RPN2610 & RPN 2617, respectively).

Paraoxonase-1 (PON-1) is a HDL-associated antioxidant enzyme with paraoxonase, arylesterase and dyazoxonase activities. For this protein, the actual enzymatic activity was analyzed according to the protocol described by Verit et al (5). Paraoxon was used as a substrate and enzymatic activity was measured by increases in the absorbance at 412 nm due to the formation of 4-nitrophenol.

PON-1 activity was determined by using paraoxon as the substrate and measured by increases in the absorbance at 412 nm due to the formation of 4-nitrophenol as already described (Isik et al. Paraoxonase and aryl-esterase levels in rheumatoid arthritis. Clin Rheumatol 2007;26: 342-348, 2007). Briefly, the activity was measured at 25°C by adding 50 ml of the serum to be tested to 1 ml Tris-HCI buffer (100 mM at pH 8.0) containing 2 mM of $CaCl_2$ and 5.5 mM of paraoxon. The rate of generation of 4-nitrophenol was determined at 412 nm. Enzymatic activity was calculated using the molar extinction coefficient 17100 $M^{-1}$ $cm^{-1}$. The *intra* and *inter*-assay coefficients of variation (CV) for PON-1 activity were <3%.

**Statistics**

The diagnostic power of the biomarkers and combinations thereof was evaluated based on the pre-laparoscopy serum levels and activity in the pain-symptomatic women with and without lesions. Predictive modeling (classifier construction) was performed to asses the diagnostic ability of different biomarker combinations. This was done by means of logistic regression. The effectiveness of the constructed classifier was evaluated with a ten-fold cross-validation. The cross-validation was repeated 100 times to get an robust result for the area under the receiver operating characteristics curve (AUC), sensitivity, specificity and accuracy for the different proteins and combinations of proteins. The logistic regression model models the probability for a binary outcome endometriosis yes/no given the observed biomarker values

$$P(Y = 1|x) = \frac{1}{1+\exp\left(-(b_0+x^Tb)\right)},$$

(levels) $x = (x_1,x_2,...,x_p)^T$ by where $b = (b_1,b_2,...,b_p)^T$ is the vector of the respective biomarkers weighing factors and $b_0$ is an additional model parameter (intercept)... The model parameters $b_0$ and $b$ were optimized using the information from the given subject population about the lesion presence and the measured biomarker values. Increased lesion probability is indicative for the presence of endometriosis in the subject.

**Results**

The biomarkers were analyzed on up to four different time point in each subject: during the proliferative phase on day 1 (up to day 4), and 9 (+/- 1d), and in the secretory phase of the menstrual cycle on days LH+3 (i.e. 3 days after the urinary LF surge) and LH+11 (i.e. 11 days after the urinary LF surge, late secretory).

For $\beta$2M protein levels as a single biomarker, performance of the test was best on days 1 to 4 and 8 to 10 of the cycle.

The classifiers comprising the combinations according to the inventions performed best on day 1 to 4 of menstrual cycle.

The diagnostic power of the proteins was evaluated based on the pre-laparoscopy serum levels in the pain-symptomatic women with and without lesions. A ten-fold cross-validation repeated 100 times was used to calculate the area under the receiver operating characteristics curve (AUC), sensitivity, specificity and accuracy for the different biomarkers and combinations thereof.

Surprisingly, β2M protein levels have been found to be decreased in pain symptomatic women with lesions as compared to those without lesions, demonstrating that decreased β2M protein levels are indicative for the presence of endometriosis in subjects with pelvic pain.

The classification performance of β2M was as follows:

Sensitivity and specificity was calculated for three cut-offs first the cut-off referred to as Youden index it is the cut-off where the sum of sensitivity and specificity is maximized and also the two cut-offs where for sensitivity or specificity at least 80% will be reached.

At day 1 to 4.

AUC: 0.56;

| Cut off definition | β2M cut | sensitivity | specificity |
|---|---|---|---|
| youden | 1.0 | 0.55 | 0.53 |
| sensitivity ≥ 80% | 1.1 | 0.80 | 0.24 |
| specificity ≥ 80% | 0.88 | 0.35 | 0.82 |

At day 8 to 10.

AUC: 0.62;

| Cut off definition | β2M cut | sensitivity | specificity |
|---|---|---|---|
| youden | 1.0 | 0.62 | 0.35 |
| sensitivity ≥ 80% | 1.3 | 0.87 | 0.12 |
| specificity ≥ 80% | 0.88 | 0.19 | 0.82 |

Further surprisingly, a set of biomarkers was identified within the group of patients suffering from pelvic pain that distinguished women with endometriosis from women with no lesions detected at time of surgery in the consecutive cycle. It turned out that these classifiers had the best diagnostic value for endometriosis in samples taken on days 1 to 4 of the menstrual cycle. Different combinations compose of 2 to 5 different markers including CA125 protein level, CA19-9 level, PON-1 activity, MMP1 protein level, MMP2 protein level and β2M protein level turned out to have predictive value, while all combinations contained β2M protein level. The predictive performance of the combinations was validated internally by repeated cross-validation runs as described above. An exemplary receiver operating curve is illustrated in Figure 1. The AUC was 0.83 for this combination with accuracy of 0.77 (sensitivity was 0.74, specificity 0.78) for the cut off defind by the Youden index (cut off where the sum of sensitivity and specitivity minus one is maximized). The individual combinations differ in achieved sensitivity and specificity with highest accuracy of 0.87 for the combination of CA125 protein level, CA19-9 level, PON1 activity, β2M protein level, and MMP2 protein level (see Table 3).

**Table 3: Results with preferred classifiers comprising combinations of biomarkers according to the invention**

Combination 1:

**AUC:** 0.75; **Sensitivity:** 0.63; **Specificity:** 0.84

Intercept ($b_0$): 2.8

| Biomarker | Weighing factor ($b_1$ to $b_p$) |
|---|---|
| CA19-9 | 0.21 |
| β2M protein | -3.0 |

(continued)

| Combination 2: **AUC:** 0.79; **Sensitivity:** 0.62; **Specificity:** 0.92 Intercept ($b_0$): 3.9 | |
|---|---|
| **Biomarker** | **Weighing factor ($b_1$ to $b_p$)** |
| CA19-9 | 0.33 |
| β2M protein | -4.6 |
| CA-125 protein | 0.015 |

| Combination 3: **AUC:** 0.78; **Sensitivity:** 0.71; **Specificity:** 0.82 Intercept ($b_0$): 2.0 | |
|---|---|
| **Biomarker** | **Weighing factor ($b_1$ to $b_p$)** |
| PON1 activity | 0.0047 |
| CA19-9 | 0.20 |
| β2M protein | -3.2 |

| Combination 4: **AUC:** 0.81; **Sensitivity:** 0.75; **Specificity:** 0.84 Intercept ($b_0$): 6.5 | |
|---|---|
| **Biomarker** | **Weighing factor ($b_1$ to $b_p$)** |
| MMP1 protein | 0.18 |
| CA19-9 | 0.36 |
| β2M protein | -7.8 |

| Combination 5: **AUC:** 0.79; **Sensitivity:** 0.87; **Specificity:** 0.66 Intercept ($b_0$): 3.8 | |
|---|---|
| **Biomarker** | **Weighing factor ($b_1$ to $b_p$)** |
| MMP2 protein | -0.00089 |
| PON1 activity | 0.0050 |
| CA19-9 | 0.22 |
| β2M protein | -3.7 |

| Combination 6: **AUC:** 0.85; **Sensitivity:** 0.76; **Specificity:** 0.94 Intercept ($b_0$): 15 | |
|---|---|
| **Biomarker** | **Weighing factor ($b_1$ to $b_p$)** |
| MMP1 protein | 0.66 |
| CA19-9 | 1.1 |
| β2M protein | -19 |
| CA-125 protein | -0.012 |
| (Intercept) | 15 |

(continued)

Combination 7:
**AUC:** 0.82; **Sensitivity:** 0.72; **Specificity:** 0.87
Intercept ($b_0$): 6.3

| Biomarker | Weighing factor ($b_1$ to $b_p$) |
|---|---|
| MMP2 protein | -0.00077 |

| Biomarker | Weighing factor ($b_1$ to $b_p$) |
|---|---|
| CA19-9 | 0.38 |
| β2M protein | -5.8 |
| CA-125 protein | 0.013 |

Combination 8:
**AUC:** 0.83; **Sensitivity:** 0.74; **Specificity:** 0.88
Intercept ($b_0$): 2.87

| Biomarker | Weighing factor ($b_1$ to $b_p$) |
|---|---|
| PON1 activity | 0.0093 |
| CA19-9 | 0.335 |
| β2M protein | -5.28 |
| CA-125 protein | 0.0166 |

Combination 9:
**AUC:** 0.67; **Sensitivity:** 0.91; **Specificity:** 0.65
Intercept ($b_0$): 16

| Biomarker | Weighing factor ($b_0$ to $b_p$) |
|---|---|
| MMP2 protein | -0.0010 |
| MMP1 protein | 0.33 |
| CA19-9 | 1.2 |
| β2M protein | -18 |
| CA-125 protein | -0.023 |

Combination 10:
**AUC:** 0.81; **Sensitivity** :0.88; **Specificity**: 0.78
Intercept ($b_0$): 5.4

| Biomarker | Weighing factor ($b_1$ to $b_p$) |
|---|---|
| MMP2 protein | -0.0011 |
| PON1 activity | 0.011 |

| Biomarker | Weighing factor ($b_1$ to $b_p$) |
|---|---|
| CA19-9 | 0.42 |
| β2M protein | -6.4 |
| CA-125 protein | 0.011 |

Combination 11:
**AUC:** 0.80; **Sensitivity:** 0.78; **Specificity:** 0.87
Intercept ($b_0$): 21

| Biomarker | Weighing factor ($b_1$ to $b_p$) |
|---|---|
| MMP1 protein | 0.61 |
| PON1 activity | -0.0055 |

(continued)

Combination 11:

**AUC:** 0.80; **Sensitivity:** 0.78; **Specificity:** 0.87

Intercept ($b_0$): 21

| Biomarker | Weighing factor ($b_1$ to $b_p$) |
| --- | --- |
| CA19-9 | 1.4 |
| $\beta$2M protein | -25 |
| CA-125 protein | -0.017 |

Combination 12:

**AUC:** 0.67; **Sensitivity:** 0.90; **Specificity:** 0.66

Intercept ($b_0$): 20

| Biomarker | Weighing factor ($b_1$ to $b_p$) |
| --- | --- |
| MMP2 protein | -0.00095 |
| MMP1 protein | 0.35 |
| PON1 activity | -0.0031 |
| CA19-9 | 1.3 |
| $\beta$2M protein | -21 |
| CA-125 protein | -0.019 |

**Discussion**

For accurate performance of the diagnostic test, the use of $\beta$2-Microglobulin protein levels as a biomarker was necessary. Performance could be improved by using a combination of multiple analytes including $\beta$2-Microglobulin protein levels at a dedicated time point in the menstrual cycle.

For CA-125 and CA-19-9, higher concentrations were described in multiple studies in endometriosis patient (1). However, there are also conflicting data suggesting no regulation in endometriosis. The inventors no found that both markers are highly regulated throughout the menstrual cycle pointing to the now found fact that the menstrual cycle if investigating those parameters.

Surprisingly, the diagnostic power of the test was highest on and before day 9 of the menstrual cycle, and of particular diagnostic power for the combinations on day 1 to 4of the menstrual cycle, which means that the first 9 days and in particular days 1 to 4 of the cycle can be used for diagnosis of endometriosis. Longitudinal data from more than 100 subjects revealed that ovarian and endometrial changes apparently affect the appearance and concentrations of multiple biomarkers in the circulation.

Importantly, the concentrations of $\beta$2M are of utmost importance for the performance of the classifiers. In contrast to the other analytes, serum $\beta$2M has not been previously associated with the presence of endometriosis. To the contrary, $\beta$2M mRNA levels were discussed as a so called housekeeping gene for quantitative real-time PCR (qPCR) of endometriotic vs. healthy tissue.

Interestingly, PON-1 analysis revealed higher levels in symptomatic women with endometriosis while reduced activity was observed in pain-free endometriosis patients compared to respective controls. Verit et al. (5) described reduced activity in endometriosis patients (age < 35y, pain in 10% of women, infertility in 58%). A more recent study found no significant changes in PON-1 activity in infertile endometriosis patients investigating 80 women in total (6). There is no data published on PON-1 activity in endometriosis patients suffering from chronic pelvic pain.

**References:**

1. Nisenblat V, Bossuyt PM, Shaikh R, Farquhar C, Jordan V, Scheffers CS, et al. Blood biomarkers for the non-invasive diagnosis of endometriosis. Cochrane Database Syst Rev. 2016 May 01 (5):CD012179.

2. Gupta D, Hull ML, Fraser I, Miller L, Bossuyt PM, Johnson N, et al. Endometrial biomarkers for the non-invasive diagnosis of endometriosis. Cochrane Database Syst Rev. 2016 Apr 20;4:CD012165.

3. Nisenblat V, Bossuyt PM, Farquhar C, Johnson N, Hull ML. Imaging modalities for the non-invasive diagnosis of endometriosis. Cochrane Database Syst Rev. 2016 Feb 26;2:CD009591.

4. Nisenblat V, Prentice L, Bossuyt PM, Farquhar C, Hull ML, Johnson N. Combination of the non-invasive tests for the diagnosis of endometriosis. Cochrane Database Syst Rev. 2016 Jul 13;7:CD012281.

5. Verit FF, Erel O, Celik N. Serum paraoxonase-1 activity in women with endometriosis and its relationship with the stage of the disease. Hum Reprod. 2008 Jan;23(1):100-4.

6. Bragatto FB, Barbosa CP, Christofolini DM, Peluso C, dos Santos AA, Mafra FA, et al. There is no relationship between Paraoxonase serum level activity in women with endometriosis and the stage of the disease: an observational study. Reprod Health. 2013 Jun 22;10:32.

**Claims**

1. A method for diagnosis of endometriosis in a subject, comprising the steps of

    (i) determining the level of β2-Microglobulin protein (β2M) as a biomarker in a sample of said subject to be diagnosed;
    (ii) comparing the biomarker level as determined in steps (i) to a respective control level derived from one or more subject without endometriosis;

    wherein a reduced level as compared to the respective control level of β2M is indicative for the presence of endometriosis in said subject to be diagnosed.

2. The method according to claim 1, wherein the sample is a serum sample, and the control level is 1.3 μg/mL or less.

3. The method according to claim 1, wherein the method further comprises

    (iii) determining the level of CA19-9 as a second biomarker in said sample of said subject to be diagnosed,
    (iv) comparing the second biomarker level as determined in steps (iii) to a respective control level derived from one or more subject without endometriosis;

    wherein an increased level as compared to the respective control level of CA 19-9 is indicative for the presence of endometriosis in said subject to be diagnosed.

4. The method according to claim 3, wherein in step (iii) the level of one or more further biomarker selected from the group consisting of CA125 protein level, PON-1 activity, MMP1 protein level and MMP2 protein level is determined and in step (iv) compared to a respective control level derived from one or more subject without endometriosis.

5. The method according to any one of claims 1 and 2 to 3, wherein the biomarkers are comprised in a diagnostic classifier.

6. The method according to claim 5, wherein the comparison of the levels of said biomarkers of the diagnostic classifier with the respective control levels is performed by calculating the probability for endometriosis using a logit model.

7. The method according to claim 6, wherein a probability of 0.6 or more is indicative for the presence of endometriosis in said subject to be diagnosed, preferably of 0.65 or more, more preferably of 0.8 or more.

8. The method according to claim 6 or 7, wherein the probability for the presence of endometriosis (in the following referred to as $P(Y = 1|x)$) is calculated with the logistic model based on the determined biomarker levels $x$ by

$$P(Y = 1|x) = \frac{1}{\left(1 + \exp(-b_0 - x^T b)\right)};$$

wherein b is the vector of weighing factors ($b = (b_1,...,b_p)^T$) for the determined biomarkers;
wherein x is the vector of the determined levels for the respective biomarkers($x = (x_1,...,x_p)^T$);
wherein $p$ is the number of determined biomarkers within the combination; and
wherein $b_0$ is the intercept in the model for the specific combination of biomarkers.

9. The method according to any one of claims 5 to 8, wherein the diagnostic classifier comprises 3 or more of the biomarkers, preferably 4 or more, yet more preferred 5 or more.

10. The method according to claim 9, wherein the diagnostic classifier comprises a combination of biomarker selected from the group combinations consisting of
β 2M protein level and CA 19-9 level (combination 1);
β 2M protein level, CA-125 protein level and CA 19-9 level (combination 2);
β 2M protein level, PON-1 activity and CA 19-9 level (combination 3);
β 2M protein level, CA 19-9 level and MMP1 protein level (combination 4);
β 2M protein level, CA 19-9 level, PON-1 activity and MMP2 protein level (combination 5);
β 2M protein level, CA-125 protein level, CA 19-9 level and MMP1 protein level (combination 6);
β 2M protein level, CA-125 protein level, CA 19-9 level and MMP2 protein level (combination 7);
β 2M protein level, CA-125 protein level, CA 19-9 level and PON-1 activity (combination 8);
β 2M protein level, CA-125 protein level, CA 19-9 level, MMP1 protein level and MMP2 protein level (combination 9);
β 2M protein level, CA-125 protein level" CA 19-9 level, PON-1 activity and MMP2 protein level (combination 10);
β 2M protein level, CA-125 protein level,CA 19-9 level, PON-1 activity and MMP1 protein level (combination 11); and
β 2M protein level, CA-125 protein level,CA 19-9 level, PON-1 activity, MMP1 protein level and MMP2 protein level (combination 12).

11. The method according to claim 10, wherein weighing factors for the respective biomarker within the specific combinations are as follows

Combination 1:

|  | Intercept ($b_0$): 2.8 |
| --- | --- |
| **Biomarker** | **Weighing factor ($b_1$ to $b_p$)** |
| CA19-9 | 0.21 |
| β2M protein | -3.0 |

Combination 2:

|  | Intercept ($b_0$): 3.9 |
| --- | --- |
| **Biomarker** | **Weighing factor ($b_1$ to $b_p$)** |
| CA19-9 | 0.33 |
| β2M protein | -4.6 |
| CA-125 protein | 0.015 |

Combination 3:

|  | Intercept ($b_0$): 2.0 |
| --- | --- |
| **Biomarker** | **Weighing factor ($b_1$ to $b_p$)** |
| PON1 activity | 0.0047 |
| CA19-9 | 0.20 |
| β2M protein | -3.2 |

Combination 4:

Intercept ($b_0$): 6.5

| Biomarker | Weighing factor ($b_1$ to $b_p$) |
|---|---|
| MMP1 protein | 0.18 |
| CA19-9 | 0.36 |
| β2M protein | -7.8 |

Combination 5:

Intercept ($b_0$): 3.8

| Biomarker | Weighing factor ($b_1$ to $b_p$) |
|---|---|
| MMP2 protein | -0.00089 |
| PON1 activity | 0.0050 |
| CA19-9 | 0.22 |
| β2M protein | -3.7 |

Combination 6:

Intercept ($b_0$): 15

| Biomarker | Weighing factor ($b_1$ to $b_p$) |
|---|---|
| MMP1 protein | 0.66 |
| CA19-9 | 1.1 |
| β2M protein | -19 |
| CA-125 protein | -0.012 |
| (Intercept) | 15 |

Combination 7:

Intercept ($b_0$): 6.3

| Biomarker | Weighing factor ($b_1$ to $b_p$) |
|---|---|
| MMP2 protein | -0.00077 |
| CA19-9 | 0.38 |
| β2M protein | -5.8 |
| CA-125 protein | 0.013 |

Combination 8:

Intercept ($b_0$): 2.87

| Biomarker | Weighing factor ($b_1$ to $b_p$) |
|---|---|
| PON1 activity | 0.0093 |
| CA19-9 | 0.335 |
| β2M protein | -5.28 |
| CA-125 protein | 0.0166 |

Combination 9:

Intercept ($b_0$): 16

| Biomarker | Weighing factor ($b_0$ to $b_p$) |
|---|---|
| MMP2 protein | -0.0010 |

(continued)

| Biomarker | Weighing factor ($b_0$ to $b_p$) |
|---|---|
| MMP1 protein | 0.33 |
| CA19-9 | 1.2 |
| β2M protein | -18 |
| CA-125 protein | -0.023 |

Combination 10:

Intercept ($b_0$): 5.4

| Biomarker | Weighing factor ($b_1$ to $b_p$) |
|---|---|
| MMP2 protein | -0.0011 |
| PON1 activity | 0.011 |
| CA19-9 | 0.42 |
| β2M protein | -6.4 |
| CA-125 protein | 0.011 |

Combination 11:

Intercept ($b_0$): 21

| Biomarker | Weighing factor ($b_1$ to $b_p$) |
|---|---|
| MMP1 protein | 0.61 |
| PON1 activity | -0.0055 |
| CA19-9 | 1.4 |
| β2M protein | -25 |
| CA-125 protein | -0.017 |

Combination 12:

Intercept ($b_0$): 20

| Biomarker | Weighing factor ($b_1$ to $b_p$) |
|---|---|
| MMP2 protein | -0.00095 |
| MMP1 protein | 0.35 |
| PON1 activity | -0.0031 |
| CA19-9 | 1.3 |
| β2M protein | -21 |
| CA-125 protein | -0.019 |

wherein the respective level of the biomarker are given with the numerical value as determined in the following units:

the levels of β2-Microglobulin protein are preferably determined and used within the method as μg/mL, more preferably as μg/mL in whole blood, more preferably as μg/mL in serum;
the levels of CA19-9 are preferably determined and used within the method as U/mL, more preferably as U/mL in whole blood, more preferably as U/mL in serum;
the levels of CA-125 are preferably determined and used within the method as U/mL, more preferably as U/mL in whole blood, more preferably as U/mL in serum;
the levels of PON-1 activity are preferably determined and used within the method as mmol/min/L, more preferably as mmol/min/L in whole blood, more preferably as mmol/min/L in serum;
the levels of MMP-1 are preferably determined and used within the method as ng/mL, more preferably as ng/mL in whole blood, more preferably as ng/mL in serum; and
the levels of MMP-2 are preferably determined and used within the method as ng/mL, more preferably as ng/mL in whole blood, more preferably as ng/mL in serum.

12. The method according to any one of claims 1 to 11, wherein the sample is collected from said subject to be diagnosed in the first ten days of the menstrual cycle, preferably in the first week of the menstrual cycle, more preferably on day 1 to 4 of the menstrual cycle.

13. The method according to any one of claims 1 to 12, wherein said subject to be diagnosed suffers from chronic pelvic pain, dysmenorrhea, dyspareunia and/or infertility.

14. The method according to any one of claims 1 to 13, wherein the biomarker protein levels are detected in an immunoassay.

15. The method according to claim 14, wherein the immunoassay is selected from the group of immunoprecipitation, enzyme immunoassay (EIA), radioimmunoassay (RIA), enzyme-linked immunosorbent assay (ELISA), fluorescent Immunoassay, a chemiluminescent assay, an agglutination assay, nephelometric assay, turbidimetric assay, a Western Blot, a competitive immunoassay, a noncompetitive immunoassay, a homogeneous immunoassay a heterogeneous immunoassay, a bioassay and a reporter assay such as a luciferase assay.

16. Use of $\beta$2-Microglobulin ($\beta$2M) binding antibody or an antigen binding peptide fragment thereof for the diagnosis of endometriosis or monitoring the response of a subject to the treatment for endometriosis.

17. The use according to claim 17, wherein the use further comprises the use of and one or more antibodies or an antigen binding peptide fragment thereof binding one of more further biomarkers selected from the group consisting of CA125, CA19-9, PON-1 activity, MMP1, and MMP2 for the diagnosis of endometriosis or monitoring the response of a subject to the treatment for endometriosis.

18. The use according to claim 18, wherein the use comprises the use of three or more antibodies or antibody binding peptide fragments thereof binding three or more of said further biomarkers.

Fig. 1

Fig. 2

Day 1 samples of cycle 1

Day 1 samples of cycle 1

Day 1 samples of cycle 1

Legend:
— B2M,CA 19-9,CA 125,MMP1,MMP2
– – B2M,CA 19-9,CA 125,PON-1,MMP2
···· B2M,CA 19-9,CA 125,PON-1,MMP1
–·– B2M,CA 19-9,CA 125,PON-1,MMP1,MMP2

False positive rate

True positive rate

Fig. 3

Beta_2_Microglobulin

Fig 4

**Cross validated AUC (100 times repeated)**
**Day 9 samples of Cycle 1**

|  | B2M |
|---|---|
| mean cv AUC : | 0.615 |
| median cv AUC : | 0.615 |

Europäisches
Patentamt

European
Patent Office

Office européen
des brevets

# EUROPEAN SEARCH REPORT

Application Number

EP 18 17 4414

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anne L Mounsey ET AL: "Diagnosis and management of endometriosis", American family physician, 15 August 2006 (2006-08-15), page 594, XP055493030, United States Retrieved from the Internet: URL:https://www.aafp.org/afp/2006/0815/p594.pdf * page 596, right-hand column, paragraph 1 - page 597, right-hand column, paragraph 1 * * abstract * | 1-18 | INV. G01N33/68 |
| A | US 2012/171694 A1 (MANSFIELD BRIAN C [US] ET AL) 5 July 2012 (2012-07-05) * claims 1-6 * | 1-18 | |
| A | K. E. MAY ET AL: "Peripheral biomarkers of endometriosis: a systematic review", HUMAN REPRODUCTION UPDATE, vol. 16, no. 6, 1 November 2010 (2010-11-01), pages 651-674, XP55187792, ISSN: 1355-4786, DOI: 10.1093/humupd/dmq009 * the whole document * | 1-18 | |
| A | STEFANO LUISI ET AL: "Serum Markers for the Noninvasive Diagnosis of Endometriosis", WOMEN'S HEALTH, vol. 11, no. 5, 1 September 2015 (2015-09-01), pages 603-610, XP055492847, UK ISSN: 1745-5057, DOI: 10.2217/whe.15.46 * abstract; table 3 * | 1-18 | |

**TECHNICAL FIELDS SEARCHED (IPC)**

G01N

The present search report has been drawn up for all claims

| Place of search | Date of completion of the search | Examiner |
|---|---|---|
| Munich | 17 July 2018 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                    
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 4414

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2018

| Patent document cited in search report | Publication date | Patent family member(s) | Publication date |
|---|---|---|---|
| US 2012171694 A1 | 05-07-2012 | US 2012171694 A1<br>US 2013267439 A1<br>US 2014364341 A1 | 05-07-2012<br>10-10-2013<br>11-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FRISHMAN GN ; SALAK JR.** *J Minim Invasive Gynecol.,* November 2006, vol. 13 (6), 546-58
- **GÜSSOW D ; REIN R ; GINJAAR I ; HOCHSTENBACH F ; SEEMANN G ; KOTTMAN A ; PLOEGH HL.** The human beta 2-microglobulin gene. Primary structure and definition of the transcriptional unit. *J. Immunol.,* 01 November 1987, vol. 139 (9), 3132-8
- **CUNNINGHAM BA ; WANG JL ; BERGGÅRD I ; PETERSON PA.** The complete amino acid sequence of beta 2-microglobulin. *Biochemistry.,* November 1973, vol. 12 (24), 4811-2
- **GARTH R. et al.** Gene: a gene-centered information resource at NCBI. *Nucleic Acids Res.,* 28 January 2015, vol. 43, D36-D42
- **MAGNANI, JL.** The discovery, biology, and drug development of sialyl Lea and sialyl Lex. *Archives of Biochemistry and Biophysics,* 15 June 2004, vol. 426 (2), 122-31
- Cancer Markers. **HOON H. SUNWOO ; MAVANUR R. SURESH.** The Immunoassay Handbook. Elsevier, 2013, 833-856
- **YIN BW ; LLOYD KO.** Molecular cloning of the CA125 ovarian cancer antigen: identification as a new mucin, MUC16. *The Journal of Biological Chemistry,* July 2001, vol. 276 (29), 27371-5
- **YIN BW ; DNISTRIAN A.** *Lloyd KO,* April 2002
- Ovarian cancer antigen CA125 is encoded by the MUC16 mucin gene. *International Journal of Cancer. Journal International Du Cancer,* vol. 98 (5), 737-40
- **DURAISAMY S ; RAMASAMY S ; KHARBANDA S ; KUFE D.** Distinct evolution of the human carcinoma-associated transmembrane mucins, MUC1, MUC4 AND MUC16. *Gene,* May 2006, vol. 373, 28-34
- **KLUG, T.L. et al.** *Cancer Res.,* 1984, vol. 44, 1048
- **VERIT FF ; EREL O ; CELIK N.** Serum paraoxonase-1 activity in women with endometriosis and its relationship with the stage of the disease. *Hum Reprod.,* January 2008, vol. 23 (1), 100-4
- **BRINCKERHOFF CE et al.** Molecular cloning of human synovial cell collagenase and selection of a single gene from genomic DNA. *J. Clin. Invest.,* February 1987, vol. 79 (2), 542-6
- **PENDAS AM et al.** Fine physical mapping of the human matrix metalloproteinase genes clustered on chromosome 11 q22.3. *Genomics,* October 1996, vol. 37 (2), 266-8
- **DEVARAJAN P et al.** Structure and expression of neutrophil gelatinase cDNA. Identity with type IV collagenase from HT1080 cells. *J. Biol. Chem.,* December 1992, vol. 267 (35), 25228-32
- **VANDER BORGHT M ; WYNS C.** Fertility and infertility: Definition and epidemiology. *Clin Biochem.,* 16 March 2018
- The Immunoassay Handbook. Elsevier LTD, May 2005
- **HULTSCHIG C et al.** *Curr Opin Chern Bioi,* February 2006, vol. l0 (1), 4-10
- **BIRD R. E. et al.** *Science,* 1988, vol. 242, 423-6
- **HOLLIGER P. et al.** *Proc. Natl. Acad. Sci. U.S.A.,* 1993, vol. 90, 6444-8
- **ISIK et al.** Paraoxonase and aryl-esterase levels in rheumatoid arthritis. *Clin Rheumatol,* 2007, vol. 26, 342-348
- **NISENBLAT V ; BOSSUYT PM ; SHAIKH R ; FARQUHAR C ; JORDAN V ; SCHEFFERS CS et al.** Blood biomarkers for the non-invasive diagnosis of endometriosis. *Cochrane Database Syst Rev.,* 01 May 2016
- **GUPTA D ; HULL ML ; FRASER I ; MILLER L ; BOSSUYT PM ; JOHNSON N et al.** Endometrial biomarkers for the non-invasive diagnosis of endometriosis. *Cochrane Database Syst Rev.,* 20 April 2016
- **NISENBLAT V ; BOSSUYT PM ; FARQUHAR C ; JOHNSON N ; HULL ML.** Imaging modalities for the non-invasive diagnosis of endometriosis. *Cochrane Database Syst Rev.,* 26 February 2016
- **NISENBLAT V ; PRENTICE L ; BOSSUYT PM ; FARQUHAR C ; HULL ML ; JOHNSON N.** Combination of the non-invasive tests for the diagnosis of endometriosis. *Cochrane Database Syst Rev.,* 13 July 2016
- **VERIT FF ; EREL O ; CELIK N.** Serum paraoxonase-1 activity in women with endometriosis and its relationship with the stage of the disease. *Hum Reprod.,* January 2008, vol. 23 (1), 100-4

- **BRAGATTO FB ; BARBOSA CP ; CHRISTOFO-LINI DM ; PELUSO C ; SANTOS AA ; MAFRA FA et al.** There is no relationship between Paraoxonase serum level activity in women with endometriosis and the stage of the disease: an observational study. *Reprod Health,* 22 June 2013, vol. 10, 32